# EUROPEAN PATENT SPECIFICATION

(11) **EP 2 322 174 B1**
(45) Date of publication and mention of the grant of the patent: **23.09.2015**
(21) Application number: 10177093.1
(22) Date of filing: 09.07.1999
(51) Int. Cl.: A61K 31/41, A61K 31/44, A61K 45/06, A61P 9/00, A61P 9/12

(54) **Combined use of valsartan and calcium channel blockers for therapeutic purposes**
Kombinierte Verwendung von Valsartan und Calcium-Kanalblockern zu therapeutischen Zwecken
Utilisation combinée de valsartane et bloqueurs du canal de calcium à but thérapeutique

(30) Priority: 10.07.1998 US 113893
(43) Date of publication of application: 18.05.2011
(62) Divisional of application: 07105179.1
(73) Proprietor: Novartis Pharma AG, 4056 Basel (CH); Novartis Pharma GmbH, 1230 Wien (AT)
(72) Inventor: de Gasparo, Marc, 2842, Rossemaiso (CH); Webb, Randy Lee, Flemington, NJ 08822 (US)
(74) Representative: Warner, James Alexander

(56) References cited:
- EP-A1- 0 628 313
- WO-A1-95/24901
- WO-A2-97/49394
- US-A- 4 654 372
- US-A- 5 492 904
- COREA L ET AL: "VALSARTAN, A NEW ANGIOTENSIN II ANTAGONIST FOR THE TREATMENT OF ESSENTIAL HYPERTENSION: A COMPARATIVE STUDY OF THE EFFICACY AND SAFETY AGAINST AMLODIPINE", CLINICAL PHARMACOLOGY AND THERAPEUTICS, NATURE PUBLISHING GROUP, US, vol. 60, 1 September 1996 (1996-09-01), pages 341-346, XP008008438, ISSN: 0009-9236, DOI: DOI:10.1016/S0009-9236(96)90061-2
- FUJIMURA Y ET AL: "Antihypertensive effect of a combination of valsartan and hydrochlorothiazide, nifedipine or propranolol in spontaneously hypertensive rats", JAPANESE PHARMACOLOGY AND THERAPEUTICS 1995 JP, vol. 23, no. 12, 1995, pages 87-93, XP002640547, ISSN: 0386-3603
- SEVER P S: "CLINICAL PROFILE OF THE NOVEL ANGIOTENSIN II TYPE I BLOCKER CANDESARTAN CILEXETIL", JOURNAL OF HYPERTENSION, LIPPINCOTT WILLIAMS & WILKINS, LTD, US / UK, vol. 15, no. SUPPL. 06, 1 December 1997 (1997-12-01), pages S09-S12, XP008058911, ISSN: 0263-6352
- OLIVAN J ET AL: "[Effect of the treatment with amlodipine on left ventricular hypertrophy in hypertensive patients]", MEDLINE,, 1 November 1996 (1996-11-01), XP002582089,
- GRANIER P ET AL: "Improvement in left ventricular hypertrophy and left ventricular diastolic function following verapamil therapy in mild to moderate hypertension", MEDLINE,, 1 January 1990 (1990-01-01), XP002582090,
- MAKRILAKIS K ET AL: "New therapeutic approaches to achieve the desired blood pressure goal", CARDIOVASCULAR REVIEWS AND REPORTS, CARDIOVASCULAR REVIEWS AND REPORTS, GREENWICH, CT, US, vol. 18, no. 12, 1 December 1997 (1997-12-01), pages 10-16, XP009133487, ISSN: 0197-3118
- TARIF N ET AL: "Preservation of renal function: the spectrum of effects by calcium-channel blockers", NEPHROLOGY DIALYSIS TRANSPLANTATION, OXFORD UNIVERSITY PRESS, GB, vol. 12, no. 11, 1 November 1997 (1997-11-01), pages 2244-2250, XP002582091, ISSN: 0931-0509
- MARKHAM A ET AL: "VALSARTAN. A REVIEW OF ITS PHARMACOLOGY AND THERAPEUTIC USE IN ESSENTIAL HYPERTENSION", DRUGS, ADIS INTERNATIONAL LTD, NZ, vol. 54, no. 2, 1 August 1997 (1997-08-01) , pages 299-311, XP002051903, ISSN: 0012-6667, DOI: DOI:10.2165/00003495-199754020-00009
- BELCHER G ET AL: "Candesartan cilexetil: safety and tolerability in healthy volunteers and patients with hypertension", JOURNAL OF HUMAN HYPERTENSION, NATURE PUBLISHING GROUP, GB, vol. 11, no. suppl. 2, 1 September 1997 (1997-09-01), pages S85-S89, XP009133334, ISSN: 0950-9240
- ALLEN T J ET AL: "Effect of combination therapy (Ang II antagonist, valsartan and a calcium channel blocker) in a hypertensive model of diabetic nephropathy", AMERICAN JOURNAL OF HYPERTENSION; FIFTEENTH SCIENTIFIC MEETING OF THE AMERICAN SOCIETY OF HYPERTENSION; NEW YORK, NEW YORK, USA; MAY 16-20, 2000, ELSEVIER, vol. 13, no. 4 Part 2, 1 April 2000 (2000-04-01), page 288A, XP002582092, ISSN: 0895-7061
- PHILIPP ET AL: "Two multicenter, 8-week, randomized, double-blind, placebo-controlled, parallel-group studies evaluating the efficacy and tolerability of amlodipine and valsartan in combination and as monotherapy in adult patients with mild to moderate essential hypertension", CLINICAL THERAPEUTICS, EXCERPTA MEDICA, PRINCETON, NJ, US, vol. 29, no. 4, 1 April 2007 (2007-04-01), pages 563-580, XP022142115, ISSN: 0149-2918, DOI: DOI:10.1016/J.CLINTHERA.2007.03.018
- NEUTEL ET AL: "Combination therapy with diuretics: An evolution of understanding", AMERICAN JOURNAL OF MEDICINE, EXCERPTA MEDICA, INC, UNITED STATES, vol. 101, no. 3, 30 September 1996 (1996-09-30), pages 61S-70S, XP005065434, ISSN: 0002-9343, DOI: 10.1016/S0002-9343(96)00269-0
- "THE SIXTH REPORT OF THE JOINT NATIONAL COMMITTEE ON PREVENTION, DETECTION, EVALUATION, AND TREATMENT OF HIGH BLOOD PRESSURE", ARCHIVES OF INTERNAL MEDICINE, AMERICAN MEDICAL ASSOCIATION, CHICAGO, IL, US, vol. 157, no. 21, 1 November 1997 (1997-11-01), pages 2413-2446, XP000885619, ISSN: 0003-9926, DOI: 10.1001/ARCHINTE.157.21.2413

## Description

The present invention relates to a pharmaceutical combination composition for use in treating or preventing hypertension comprising:
(i) the AT₁-antagonist valsartan or a pharmaceutically acceptable salt thereof;
(ii) amlodipine or a pharmaceutically acceptable salt thereof; and a pharmaceutically acceptable carrier.

Valsartan is specifically and generically disclosed in EP 0443983 A. Valsartan is the AT1 receptor antagonist (S)-N-(1-carboxy-2-methyl-prop-1-yl)-N-pentanoyl-N-[2'(1H-tetrazol-5-yl)biphenyl-4-yl-methyl]amine (valsartan) of formula (I)

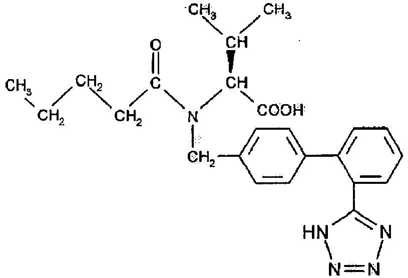

The class of CCBs essentially comprises dihydropyridines (DHPs) and non-DHPs such as diltiazem-type and verapamil-type CCBs.

The CCB used in said combination is amlodipine or a pharmaceutically acceptable salt thereof. The amlodipine may be present as a pharmaceutically acceptable salt, especially the besylate.

The valsartan and amlodipine to be combined can be present as pharmaceutically acceptable salts. If these compounds have, for example, at least one basic centre, they can form acid addition salts. Corresponding acid addition salts can also be formed having, if desired, an additionally present basic centre. The compounds having at least one acid group (for example COOH) can also form salts with bases. Corresponding internal salts may furthermore be formed, if a compound of formula comprises e.g. both a carboxy and an amino group.

A preferred salt is amlodipine besylate.

The vasoconstrictive effects of angiotensin II are produced by its action on the non-striated smooth muscle cells, the stimulation of the formation of the adrenergenic hormones epinephrine and norepinephrine as well as the increase of the activity of the sympathetic nervous system as a result of the formation of norepinephrine. Angiotensin II also has an influence on the electrolytic balance, produces e.g. antinatriuretic and antidiuretic effects in the kidney and thereby promotes the release of, on the one hand, the vasopressin peptide from the pituitary gland and, on the other hand, of aldosterone from the adrenal glomerulosa. All these influences play an important part in the regulation of blood pressure, in increasing both circulating volume and peripheral resistance. Angiotensin II is also involved in cell growth and migration and in extracellular matrix formation.

Angiotensin II interacts with specific receptors on the surface of the target cell. It has been possible to identify receptor subtypes which are termed e.g. AT₁- and AT₂-receptors. In recent times great efforts have been made to identify substances that bind to the AT₁-receptor. Such active ingredients are often termed angiotensin II antagonists. Because of the inhibition of the AT₁-receptor such antagonists can be used e.g. as antihypertensives or for the treatment of congestive heart failure.

Angiotensin II antagonists are therefore understood to be those active ingredients which bind to the AT₁-receptor subtype but do not result in activation of the receptor.

Prolonged and uncontrolled hypertensive vascular disease ultimately leads to a variety of pathological changes in target organs such as the heart and kidney. Sustained hypertension can lead as well to an increased occurrence of stroke. Therefore, there is a strong need to evaluate the efficacy of antihypertensive therapy, an examination of additional cardiovascular endpoints, beyond those of blood pressure lowering, to get further insight into the benefits of combined treatment.

The nature of hypertensive vascular diseases is multifactorial. Under certain circumstances, drugs with different mechanisms of action have been combined. However, just considering any combination of drugs having different mode of action does not necessarily lead to combinations with advantageous effects.

AT₁ antagonist and CCB reduce intracellular calcium by different and complementary mechanisms and facilitate the vasodilator effects of nitric oxide, being particularly effective in reversing endothelium dysfunction.

All the more surprising is the experimental finding that the combined administration of the AT₁-antagonist valsartan or a pharmaceutically acceptable salt thereof and a CCB or a pharmaceutically acceptable salt thereof results not only in a synergistic therapeutic effect but also in additional benefits resulting from combined treatment such as a surprising prolongation of efficacy and a broader variety of therapeutic treatment. This includes hemodynamic, renal, antiproliferative, antithrombotic and antiatherogenic properties.

The measurement of cardiac mass to assess treatment-induced regression of hypertrophy provided data to support a supra-additive effect of combination of the present invention. Left ventricular hypertrophy is an independent risk factor for the development of myocardial infarction. Thus, effective blood pressure lowering coupled with the ability to regress or prevent the development of left ventricular hypertrophy has an impact on two important and contributing factors for heart failure.

Further benefits are that lower doses of the individual drugs to be combined according to the present invention can be used to reduce the dosage, for example, that the dosages need not only often be smaller but are also applied less frequently, or can be used to diminish the incidence of side effects. This is in accordance with the desires and requirements of the patients to be treated.

It can be shown that combination therapy with valsartan and a calcium channel blocker results in a more effective antihypertensive therapy (whether for malignant, essential, reno-vascular, diabetic, isolated systolic, or other secondary type of hypertension) through improved efficacy as well as a greater responder rate.

The person skilled in the pertinent art is fully enabled to select a relevant test model to prove the hereinbefore and hereinafter indicated therapeutic indications.

Representative studies are carried out with a combination of valsartan and amlodipine, e.g. applying following methodology. All experiments are performed in spontaneously hypertensive rats (SHRs) supplied by Taconic Farms, Germantown, New York (Tac:N(SHR)fBR). A radiotelemetric device (Data Sciences International, Inc., St. Paul, Minnesota) is implanted into the lower abdominal aorta of all test animals between the ages of 14 to 16 weeks of age. All SHR are allowed to recover from the surgical implantation procedure for at least 2 weeks prior to the initiation of the experiments. The radiotransmitter is fastened ventrally to the musculature of the inner abdominal wall with a silk suture to prevent movement. Cardiovascular parameters are continuously monitored via the radiotransmitter and transmitted to a receiver where the digitized signal is then collected and stored using a computerized data acquisition system. Blood pressure (mean arterial, systolic and diastolic pressure) and heart rate are monitored in conscious, freely moving and undisturbed SHR in their home cages. The arterial blood pressure and heart rate are measured every 10 minutes for 10 seconds and recorded. Data reported for each rat represent the mean values averaged over a 24 hour period and are made up of the 144 time points of 10 minute duration samples collected each day. The baseline values for blood pressure and heart rate consist of the average of three consecutive 24 hour readings taken prior to initiating the drug treatments. All rats are individually housed in a temperature and humidity controlled room and are maintained on a 12 hour light/dark cycle.

In addition to the cardiovascular parameters, weekly determinations of body weight also are recorded in all rats. Since all treatments are administered in the drinking water, water consumption is measured five times per week. Valsartan and amlodipine doses for individual rats are then calculated based on water consumption for each rat, the concentration of drug substance in the drinking water, and individual body weights. All drug solutions in the drinking water are made up fresh every three to four days.

Upon completion of the 6 week treatment, SHR are anesthetized and the heart rapidly removed. After separation and removal of the atrial appendages, left ventricle and left plus right ventricle (total) are weighed and recorded. Left ventricular and total ventricular mass are then normalized to body weight and reported. All values reported for blood pressure and cardiac mass represent the group mean + sem.

Valsartan and amlodipine are administered via the drinking water either alone or in combination to SHR beginning at 18 weeks of age and continued for 6 weeks. Based on a factorial design, seven (7) treatment groups are used to evaluate the effects of combination therapy on blood pressure and heart rate. Treatment groups consist of Valsartan alone in drinking water at a concentration of 240 mg/liter (high dose), amlodipine alone at a concentration of 120 mg/liter (high dose), valsartan (120 mg/liter) + amlodipine (60), valsartan (120) + amlodipine (120), valsartan (240) + amlodipine (60), valsartan (240) + amlodipine (120) and a vehicle control group on regular drinking water.

Thus, 4 groups of SHR receive combination therapy.

Studies have been performed in SHR and demonstrate that the addition of a CCB confers additional benefit over that of valsartan monotherapy. The Area Under the Curve (AUC) for blood pressure reflects the changes in response to 6 week treatment in conscious SHR. Upon completion of the 6 week treatment period, hearts are removed for assessment of left ventricle mass and normalized to body weight.

The available results indicate an unexpected beneficial effect of a combination according to the invention.

It is the object of this invention to provide a pharmaceutical combination composition for the treatment or prevention of hypertension which composition comprises (i) the AT₁-antagonist valsartan or a pharmaceutically acceptable salt thereof and (ii) amlodipine or a pharmaceutically acceptable salt thereof and a pharmaceutically acceptable carrier.

In this composition, components (i) and (ii) are obtained and administered together, in one fixed combination combined unit dose form.

The corresponding active ingredient or a pharmaceutically acceptable salt thereof may also be used in form of a hydrate or include other solvents used for crystallization.

The pharmaceutical compositions for use according to the invention can be prepared in a manner known per se and are those suitable for enteral, such as oral or rectal, and parenteral administration to mammals (warm-blooded animals), including man, comprising a therapeutically effective amount of the pharmacologically active compound, alone or in combination with one or more pharmaceutically acceptable carries, especially suitable for enteral or parenteral application.

The pharmaceutical preparations contain, for example, from about 10 % to about 100 %, preferably 80%, preferably from about 20 % to about 60 %, of the active ingredient. Pharmaceutical preparations for use according to the invention for enteral or parenteral administration are, for example, those in unit dose forms, such as sugar-coated tablets, tablets, capsules or suppositories, and furthermore ampoules. These are prepared in a manner known per se, for example by means of conventional mixing, granulating, sugar-coating, dissolving or lyophilizing processes. Thus, pharmaceutical preparations for oral use can be obtained by combining the active ingredient with solid carriers, if desired granulating a mixture obtained, and processing the mixture or granules, if desired or necessary, after addition of suitable excipients to give tablets or sugar-coated tablet cores.

The determination of the dose of the active ingredients necessary to achieve the desired therapeutic effect is within the skill of those who practice in the art. The dose depends on the warm-blooded animal species, the age and the individual condition and on the manner of administration. In the normal case, an approximate daily dose in the case of oral administration for a patient weighing approximately 75 kg for oral application is of about 10 mg to about 200 mg, especially about 20 to about 120 mg, most preferably about 40 mg to about 80 mg for valsartan and about 1.0 mg to about 180 mg, preferably about 2.5 mg to about 50 mg, for the CCB.

The following example illustrates the invention described above; however, it is not intended to limit its extent in any manner.

### Valsartan Tablet Formulation 80 mg + Amlodipine 5 mg (Rollercompaction)

| | |
|---|---|
| Dosage (mg) | 80 mg Valsartan + 5 mg Amlodipine |
| Diameter (mm) | 9 |
| Shape | round |
| Breaking line | without |
| Tablet weight (mg) | 215 |

### Formulation of the Tablet Valsartan 80 mg + Amlodipine 5 mg

| | Dosage Strength | Function of the Excipient in the Formulation | 80 mg Valsartan + 5 mg Amlodipine |
|---|---|---|---|
| I. | Compactate | | mg: |
| 1. | Valsartan DS | drug substance | 80.0 |
| 2. | Amlodipine DS | drug substance | 5.0 |
| 3. | Avicel PH 102 | filler | 104.0 |
| 4. | PVPP-XL | disintegrant | 20.0 |
| 5. | Aerosil 200 | glidant | 0.75 |
| 6. | Magnesium-stearate | lubricant | 2.5 |

| II. | Outer Phase | | |
|---|---|---|---|
| 7. | Aerosil 200 | glidant | 0.75 |
| 8. | Magnesium-stearate | lubricant | 2.0 |
| | total | | 215.0 |

## Claims

1. A pharmaceutical combination composition for use in treating or preventing hypertension comprising:
(i) the AT₁-antagonist valsartan or a pharmaceutically acceptable salt thereof;
(ii) amlodipine or a pharmaceutically acceptable salt thereof, and
a pharmaceutically acceptable carrier,
wherein the combination composition is in one fixed combination combined unit dose form.

2. The pharmaceutical combination composition for use according to claim 1 wherein component (ii) is amlodipine besylate.

## Patentansprüche

1. Pharmazeutische Kombinationszusammensetzung zur Verwendung bei der Behandlung oder Vorbeugung von Bluthochdruck, umfassend
(i) den AT1-Antagonisten Valsartan oder ein pharmazeutisch unbedenkliches Salz davon;
(ii) Amlodipin oder ein pharmazeutisch unbedenkliches Salz davon und
einen pharmazeutisch unbedenklichen Trägerstoff, wobei die Kombinationszusammensetzung als in einer festen Kombination kombinierte Einheitsdosisform vorliegt.

2. Pharmazeutische Kombinationszusammensetzung zur Verwendung gemäß Anspruch 1, wobei es sich bei Komponente (ii) um Amlodipinbesylat handelt.

## Revendications

1. Composition combinée pharmaceutique destinée à l'utilisation pour le traitement ou la prévention de l'hypertension, comprenant :
(i) l'antagoniste AT₁ valsartane ou un sel pharmaceutiquement acceptable de celui-ci ;
(ii) l'amlodipine ou un sel pharmaceutiquement acceptable de celle-ci, et
un support pharmaceutiquement acceptable,
la composition combinée se présentant sous la forme d'une dose unitaire combinée en une combinaison fixe.

2. Composition combinée pharmaceutique destinée à l'utilisation selon la revendication 1, dans laquelle le composant (ii) est le bésylate d'amlodipine.
